(19) Europäisches Patentamt
European Patent Office
Office européen des brevets

(11) Veröffentlichungsnummer: **0 053 261**
**B1**

(12) # EUROPÄISCHE PATENTSCHRIFT

(45) Veröffentlichungstag der Patentschrift: **21.12.83**

(51) Int. Cl.$^3$: **C 25 B 3/02,** C 07 C 43/305 // C07C50/04

(21) Anmeldenummer: **81108482.1**

(22) Anmeldetag: **19.10.81**

(54) **Verfahren zur Herstellung eines Gemisches von Ketalen des Trimethyl-p-benzochinons.**

(30) Priorität: **02.12.80 DE 3045370**

(43) Veröffentlichungstag der Anmeldung: **09.06.82 Patentblatt 82/23**

(45) Bekanntmachung des Hinweises auf die Patenterteilung: **21.12.83 Patentblatt 83/51**

(84) Benannte Vertragsstaaten: **BE CH DE FR GB IT LI**

(56) Entgegenhaltungen: **DE - A - 2 547 383**

(73) Patentinhaber: **BASF Aktiengesellschaft, Carl-Bosch-Strasse 38, D-6700 Ludwigshafen (DE)**

(72) Erfinder: **Degner, Dieter, Dr., Kurpfalzstrasse 8, D-6701 Dannstadt-Schauernheim (DE)**
Erfinder: **Barl, Manfred, Dr., Pappelstrasse 2, D-6701 Otterstadt (DE)**

**EP 0 053 261 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung eines Gemisches von Ketalen des Trimethyl-p-benzochinons

Die vorliegende Erfindung betrifft ein neues elektrochemisches Verfahren zur Herstellung von Ketalen des Trimethyl-p-benzochinons.

Ketale des Trimethyl-p-benzochinons können z. B. durch Oxidation von Trimethylhydrochinondimethylether hergestellt werden. Dieses in J. Org. Chem. 45, 369–378 (1980) beschriebene Verfahren ermöglicht jedoch keinen attraktiven Zugang zu den Ketalen des Trimethyl-p-benzochinons, da die Hydrochinonderivate wiederum nur durch Reduktion des Trimethyl-p-benzochinons gut zugänglich sind.

Es wurde nun gefunden, daß man ein Gemisch von Ketalen des Trimethyl-p-benzochinons der Formel

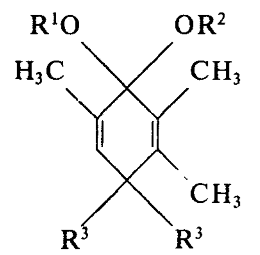

(I)

in der $R^1$ und $R^2$ Alkylgruppen mit 1 bis 4 C-Atomen und $R^3$ den $R^2O$-Rest oder beide Reste $R^3$ zusammen ein Sauerstoffatom bedeuten, besonders vorteilhaft dadurch herstellen kann, daß man Alkyltrimethylphenylether der Formel

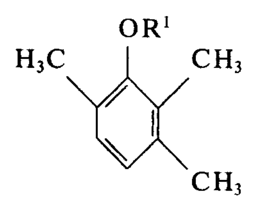

(II)

in Gegenwart eines Alkanols der Formel $R^2OH$ elektrochemisch oxidiert.

Wegen der erheblich besseren Zugänglichkeit der Alkylether des Trimethylphenols ermöglicht das neue Verfahren eine sehr vorteilhafte Herstellung von Ketalen des Trimethyl-p-benzochinons. Das erfindungsgemäße Verfahren eröffnet auch einen einfachen Weg zur Herstellung von Trimethyl-p-benzochinon. Die Reaktion wird durch folgendes allgemeine Schema wiedergegeben:

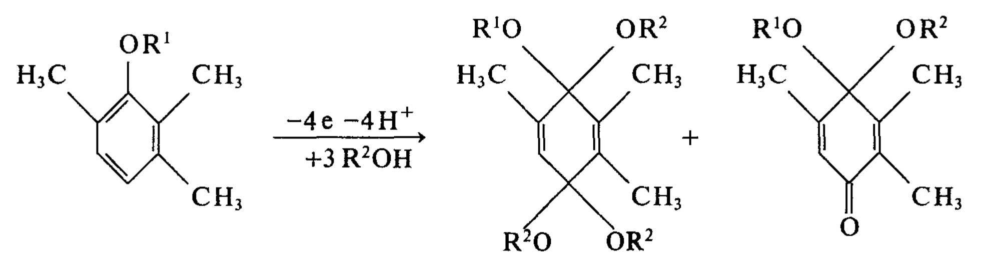


Als Alkylreste $R^1$ und $R^2$ kommen z. B. Methyl-, Ethyl- oder Propylgruppen in Betracht. Methylgruppen sind bevorzugt.

Es ist aus der DE-A-2 547 383 bekannt, daß bei der anodischen Oxidation von o- und m-Methoxytoluol vorwiegend der Benzolkern substituiert wird. Da jedoch bei der anodischen Oxidation substituierter Toluole in Methanol, wie sie in J. Chem. Soc. Perkin Trans. I, 1978, 7, Seiten 708–715 beschrieben wird, sowohl seitenkettensubstituierte als auch kernsubstituierte Verbindungen erhalten werden, und bei der Elektrooxidation einer Benzolverbindung mit zwei Methylgruppen in Methanol Seitenkettenoxidation eintritt (s. DE-PS 2 848 397, Beispiel 8) war nicht zu erwarten, daß beim erfindungsgemäßen Verfahren praktisch keine Seitenkettenoxidation bei den drei Methylgruppen stattfindet.

Die als Ausgangsstoffe für die Elektrosynthese benötigten Alkylether des Trimethylphenols lassen sich auf einfache Weise, z. B. durch Umsetzung von Trimethylphenol mit Alkylsulfaten herstellen. So führt z. B. die Umsetzung von 2,3,6-Trimethylphenol mit Dimethylsulfat in über 90%iger Ausbeute zu 1-Methoxy-2,3,6-trimethylbenzol. Die Elektrosynthese erfordert keine besonders gereinigten Produkte, so kann z. B. das Rohprodukt der Umsetzung von 2,3,6-Trimethylphenol mit Dimethylsulfat direkt in die Elektrosynthese eingesetzt werden.

Das neue elektrochemische Verfahren kann sowohl in geteilten als auch in ungeteilten Zellen

durchgeführt werden. Aus technischen Gründen werden ungeteilte Zellen bevorzugt, da sie geringe Elektrodenabstände und somit einen geringeren Energieaufwand ermöglichen. Als Elektrodenmaterialien werden z. B. Edelmetallelektroden wie Platin eingesetzt. Bevorzugtes Elektrodenmaterial ist Graphit. Als Elektrolyt wird eine Lösung des Alkyl-trimethylethers in dem Alkanol, wie Methanol oder Ethanol oder in einem Lösungsmittel eingesetzt, das das Alkanol enthält. Vorteilhaft enthält der Elektrolyt ein Leitsalz. Als Leitsalze werden bevorzugt Fluoride wie KF, Tetrafluoroborate wie $(C_4H_9)_4N^{\oplus}BF_4^{\ominus}$, Sulfonate wie

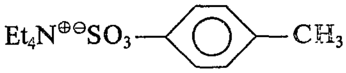

oder Sulfate wie $Et_4N^{\oplus}SO_4Et^{\ominus}$ verwendet. Der Elektrolyt setzt sich beispielsweise wie folgt zusammen:

| | |
|---|---|
| 5 – 50 Gew.-% | Alkyl-trimethylphenyl-ether |
| 50 – 90 Gew.-% | Alkanol |
| 0,1 – 10 Gew.-% | Leitsalz |

Das Verfahren wird im allgemeinen bei Stromdichten von 1 bis 25 A/dm² und bei Temperaturen bis etwa 5° C unterhalb des Siedepunkts des Alkanols durchgeführt.

Die bei dem Verfahren erhältlichen Ketale sind wertvolle Produkte für die Synthese des Trimethyl-p-benzochinons, das seinerseits als Vorprodukt für die technische Herstellung von Vitamin E benötigt wird. Die Hydrolyse der Ketale zur Herstellung von Trimethyl-p-benzochinon kann z. B. durch Umsetzung mit Wasser in Gegenwart geringer Mengen an Säuren durchgeführt werden.

## Beispiel

| | |
|---|---|
| Apparatur: | ungeteilte Zelle mit 11 Elektroden |
| Elektrodenabstand: | 0,5 mm |
| Anoden: | Graphit, Fläche pro Anode: 1,5 dm² |
| Elektrolyt: | 294 g 1-Methoxy-2,3,6-trimethyl-benzol<br>2370 g Methanol<br>25 g KF |
| Kathoden: | Graphit |
| Stromdichte: | 5,4 A/dm² |
| Temperatur: | 24 bis 35° C |

Elektrolyse mit 4 F/Mol 1-Methoxy-2,3,6-trimethyl-benzol. Der Elektrolyt wird während der Elektrolyse mit 200 l/h über einen Wärmetauscher gepumpt.

## Aufarbeitung

Nach Beendigung der Elektrolyse wird Methanol bei Normaldruck abdestilliert. Man filtriert das KF bei ca. 60 bis 80° C ab. Der verbleibende Rückstand wird bei 3 bis 10 mbar und 84 bis 100° C fraktioniert destilliert. Hierbei erhält man neben 71,3 g unumgesetzten 1-Methoxy-2,3,6-trimethyl-benzol, 90,6 g 1,1'-Dimethoxy-2,3,6-trimethyl-2,5-cyclohexadienon-4 und 165,2 g 1,1',4,4'-Tetramethoxy-2,3,6-trimethyl-2,5-cyclohexadien. Dies entspricht einer Gesamtketalausbeute von 77%.

**Patentansprüche**

1. Verfahren zur Herstellung eines Gemisches von Ketalen des Trimethyl-p-benzochinons der Formel

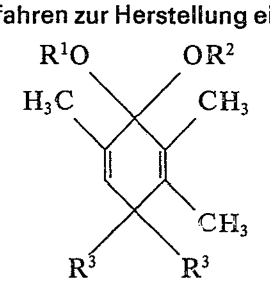

(I)

in der $R^1$ und $R^2$ Alkylgruppen mit 1 bis 4 C-Atomen und $R^3$ den $R^2O$-Rest oder beide Reste $R^3$ zusammen ein Sauerstoffatom bedeuten, dadurch gekennzeichnet, daß man einen Alkyltrimethylphenylether

der Formel

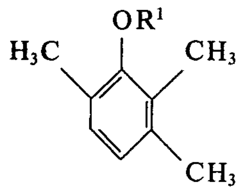

(II)

in Gegenwart eines Alkanols der Formel $R^2OH$ elektrochemisch oxidiert.

2. Verfahren nach Anspruch 1, dadurch gekennzeichnet, daß man als Alkyltrimethylphenylether der Formel II 1-Methoxy-2,3,6-trimethylbenzol verwendet.

3. Verfahren nach Anspruch 1, dadurch gekennzeichnet, daß man als Alkanol Methanol verwendet.

**Claims**

1. A process for the preparation of a mixture of ketals of trimethyl-p-benzoquinone, of the formula

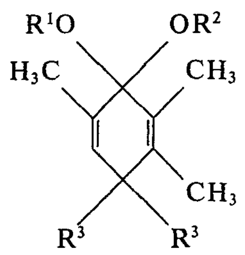

(I)

where $R^1$ and $R^2$ are alkyl of 1 to 4 carbon atoms and $R^3$ is $R^2O$, or the two radicals $R^3$ together are oxygen, wherein an alkyl trimethylphenyl ether of the formula

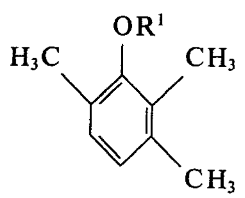

(II)

is oxidized electrochemically in the presence of an alkanol of the formula $R^2OH$.

2. A process as claimed in claim 1, wherein 1-methoxy-2,3,6-trimethylbenzene is used as the alkyl trimethylphenyl ether of the formula II.

3. A process as claimed in claim 1, wherein methanol is used as the alkanol.

**Revendications**

1. Procédé de préparation d'un mélange de cétals de la triméthyl-p-benzoquinone de la formule

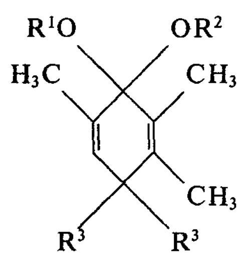

(I)

dans laquelle $R^1$ et $R^2$ désignent des radicaux alcoyle en $C_1$ à $C_4$ et $R^3$ désigne un groupe $R^2O$- ou les deux restes $R^3$ représentent ensemble un atome d'oxygène, caractérisé en ce que l'on soumet un éther alcoyl-triméthyl-phénylique de la formule

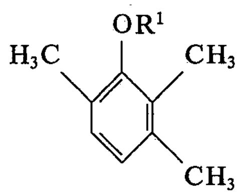

(II)

en présence d'un alcanol de la formule $R^2OH$ à une oxydation par voie électro-chimique.

2. Procédé suivant la revendication 1, caractérisé en ce que l'éther alcoyl-triméthyl-phénylique de la formule II est le méthoxy-1 triméthyl-2,3,6 benzène.

3. Procédé suivant la revendication 1, caractérisé en ce que l'alcanol est le méthanol.